(19) Europäisches Patentamt
European Patent Office
Office européen des brevets

(11) EP 2 417 962 A1

(12) **EUROPEAN PATENT APPLICATION**
published in accordance with Art. 153(4) EPC

(43) Date of publication:
**15.02.2012 Bulletin 2012/07**

(21) Application number: **10761650.0**

(22) Date of filing: **02.04.2010**

(51) Int Cl.:
***A61K 8/34*** *(2006.01)* ***A61K 8/81*** *(2006.01)*
***A61K 8/894*** *(2006.01)* ***A61K 8/898*** *(2006.01)*
***A61Q 5/06*** *(2006.01)* ***C08F 220/10*** *(2006.01)*
***C08F 290/06*** *(2006.01)*

(86) International application number:
**PCT/JP2010/056058**

(87) International publication number:
**WO 2010/116951 (14.10.2010 Gazette 2010/41)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **07.04.2009 JP 2009093244**

(71) Applicant: **Shiseido Company, Ltd.**
**Chuo-ku**
**Tokyo 104-8010 (JP)**

(72) Inventors:
- **TOYODA, Tomonori**
**Yokohama-shi**
**Kanagawa**
**2248558 (JP)**
- **SHIMIZU, Hideki**
**Yokohama-shi**
**Kanagawa**
**2248558 (JP)**
- **KURASHIMA, Takumi**
**Yokohama-shi**
**Kanagawa 224-8558 (JP)**
- **FUJIYAMA, Taizo**
**Yokohama-shi**
**Kanagawa 224-8558 (JP)**
- **MIYAZAWA, Kazuyuki**
**Yokohama-shi**
**Kanagawa 224-8558 (JP)**

(74) Representative: **Henkel, Breuer & Partner**
**Patentanwälte**
**Maximiliansplatz 21**
**80333 München (DE)**

# (54) HAIR COSMETIC

(57) Disclosed is a hair cosmetic which can achieve fixing of hair (hairstyle) by sticking and also has an excellent arranging ability (also including a restyling ability), and moreover imparts a good sense of use. Specifically disclosed is a hair cosmetic comprising a novel adhesive setting resin which is obtained by combining and polymerizing monomers having a specific structure and has a moderate firmness and a high adhesive force when forming a film and further comprising at least one member selected from a cationic surfactant, a silicone derivative, and a polyhydric alcohol, and an alcohol. Accordingly, a hair cosmetic having both fixing ability and arranging ability, and also imparting an excellent sense of use (smoothness and moistness) is obtained.



Printed by Jouve, 75001 PARIS (FR)

## Description

Technical Field

The present invention relates to a hair cosmetic that has both the abilities to set and arrange hair (also including the ability to restyle hair) and is further excellent in moisture resistance. More specifically, the present invention relates to a hair cosmetic that is capable of setting hair based on fixation and restyling the hair and is excellent in the ability to retain set hairstyles, by virtue of a novel polymer contained therein and a setting resin further incorporated therein.

Background Art

Hair styling includes two functions: forming hairstyles and keeping the formed hairstyles. The principles on which these two functions are exerted are allegedly fixation and adhesion (Non-Patent Document 1).

Hair styling based on fixation is to set hair by forming a solid film using a film-forming agent (polymer resin) called a setting agent. For example, conventional hair gels or hair sprays are based mainly on a hair styling mechanism using a setting resin. For example, Patent Document 1 discloses a hair cosmetic mainly comprising a film-forming polymer such as polyvinylpyrrolidone, sodium polyacrylate, or a polyvinylpyrrolidone-polyvinyl acetate copolymer as a setting resin. Patent Document 2 discloses a hair cosmetic comprising a silylated urethane resin as a setting resin, and has reported that this hair cosmetic forms a film having both softness and hardness and has natural textures and a high ability to keep hairstyles.

However, the hair cosmetics obtained using the resins as described in Patent Documents 1 and 2 disadvantageously fails to restyle hair from a temporarily formed hairstyle due to a hard film formed by the setting resin and loses styling functions when the film is broken. Specifically, a problem of styling agents based on fixation brought about by such a setting resin is the poor ability to arrange hair, though they are excellent in setting hair.

On the other hand, styling based on adhesion is to allow hairs to adhere to one another by an oily ingredient. Hair liquids comprising an adhesive oily ingredient such as polyalkylene glycol as a main base, hair waxes that utilize the adhesiveness of a solid oil and have been preferred by the youth in recent years, and so on are known as such styling agents. For example, Patent Document 3 discloses a cosmetic for hair that comprises waxes and a spinnable water-soluble polymer and is excellent in restyling hair.

However, hair styling based on the adhesiveness of such an oily ingredient is characterized by being capable of restyling through fingers or a brush because the oily ingredient retains flowability and adhesiveness on the hair. A problem of this hair styling is that the ability to set hair (ability to keep hairstyles) as in hair cosmetics comprising a setting resin is not obtained, though it is excellent in the so-called ability to arrange hair.

Prior Art Documents

Non-Patent Documents

Non-Patent Document 1: "Development of Advanced Cosmetics II", ed. by Masato Suzuki, published by CMC Publishing Co., Ltd., 1996, Chapter 10: Functions of Hair-styling Agents and State-of-the-Art Technology

Patent Documents

Patent Document 1: JP-A-2006-213706
Patent Document 2: JP-A-2003-171244
Patent Document 3: JP-A-Hei 10-45546

Summary of the Invention

Problems to be solved by the Invention

Accordingly, an object of the present invention is to provide a hair cosmetic that is capable of setting hair (hairstyle) based on fixation, is also excellent in the ability to arrange hair (also including the ability to restyle hair), and further imparts favorable feelings in use.

The present inventors have conducted diligent studies to attain the object and consequently completed the present invention by finding that a hair cosmetic having both the abilities to set and arrange hair is obtained by incorporating,

as a setting resin, a novel adhesive setting resin having moderate hardness and high adhesive strength during film formation, and by also finding that improved feelings in use without stickiness are achieved by further incorporating therein at least one selected from a cationic surfactant, a silicone derivative, and a polyhydric alcohol.

Means for Solving the Problems

Specifically, the present invention provides a hair cosmetic comprising:

(a) an adhesive setting resin obtained by polymerizing: at least one monomer represented by the following formula (A) (hereinafter, referred to as a "monomer A"):

[Formula 1]

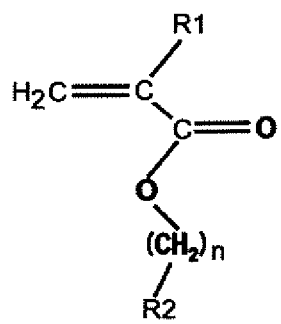

(A)

wherein R1 represents H or $CH_3$; n represents an integer of 0 to 30; $(CH_2)_n$ contains a branched chain; and R2 represents H, OH, $OCH_3$, $OCH_2CH_3$, or phenyl,
and/or
at least one monomer represented by the following formula (B) (hereinafter, referred to as a "monomer B"):

[Formula 2]

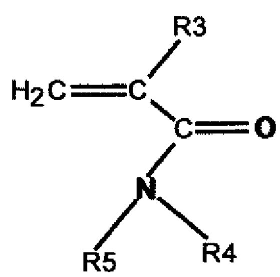

(B)

wherein R3 represents H or $CH_3$; R4 and R5, which may be the same or different, each represent H or $(CH_2)_1R'$; 1 represents an integer of 1 to 3; R' represents H, OH, or -NR"R"'; and R" and R"', which may be the same or different, each represent H or a C1-C3 alkyl group,
and
at least one monomer represented by the following formula (C) (hereinafter, referred to as a "monomer C"):

[Formula 3]

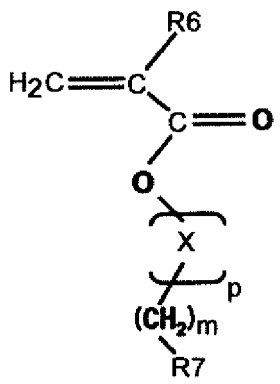

(C)

wherein R6 represents H or $CH_3$; p represents an integer of 1 to 100; m represents an integer of 0 to 30; R7 represents H, OH, $OCH_3$, $OCH_2CH_3$, or phenyl; and X represents an oxyethylene group (EO), an oxypropylene group (PO), an oxybutylene group (BO), or glyceryl,
and
at least one monomer represented by the following formula D (hereinafter, referred to as a "monomer D"):

[Formula 4]

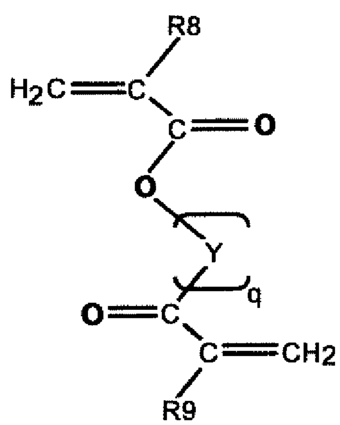

(D)

wherein R8 represents H or $CH_3$; q represents an integer of 1 to 100; and Y represents an oxyethylene group (EO), an oxypropylene group (PO), an oxybutylene group (BO), a linear or branched oxyalkylene group having 5 or more carbon atoms, or glyceryl, provided that q represents 1 when Y represents an oxyalkylene group having 5 or more carbon atoms,
(b) at least one selected from a cationic surfactant, a silicone derivative, and a polyhydric alcohol, and
(c) alcohol.

Effects of the Invention

A hair cosmetic of the present invention can have both the abilities to set and arrange hair, which are impossible to achieve for conventional setting resins, by incorporating therein the novel adhesive setting resin described above. Furthermore, the hair cosmetic of the present invention can impart improved feelings in use without stickiness by further incorporating at least one selected from a cationic surfactant, a silicone derivative, and a polyhydric alcohol, in addition to the adhesive setting resin.

Modes for Carrying out the Invention

A hair cosmetic of the present invention comprises, as an essential ingredient, an adhesive setting resin obtained by polymerizing the monomers A and/or B, and C, and D described above. Specifically, for the adhesive setting resin of the present invention, it is essential to comprise the monomers C and D. The adhesive setting resin of the present invention can comprise any one of the monomers A and B, or both. A resin (polymer) that lacks the monomer C or D produces neither favorable adhesive strength nor the ability to arrange hair (ability to restyle hair).

It is particularly preferred that the adhesive setting resin used in the present invention should have a structure represented by the following formula (I):

[Formula 5]

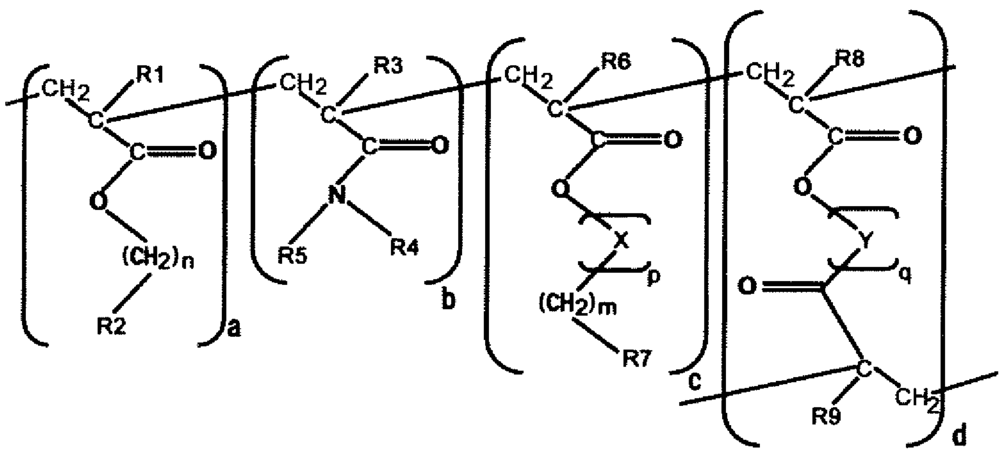

(I)

In the formula (I), R1 to R9, n, m, p, and q are as defined in the formulas A to D. The terms in the present specification are used as usual meanings. For example, an oxyethylene group (EO), an oxypropylene group (PO), and an oxybutylene group (BO) mean linear or branched oxyalkylene groups having 2, 3, and 4 carbon atoms, respectively. Moreover, in the formula (I), a represents a number within the range of $40 < a < 400$; b represents a number within the range of $80 \leq b < 300$; c represents a number within the range of 30 c < 300; and d represents a number within the range of $0 < d < 10$. The percentage by mass of each monomer in the adhesive setting resin (polymer of the formula (I)) that satisfies the conditions described above is approximately as follows: $7.5 < A < 62.5$, $20 \leq B < 45$, $7.5 < C < 60$, and $0 < D < 5$.

The adhesive setting resin of the present invention can be prepared by mixing the monomers A and/or B, and C, and D at an appropriate ratio and polymerizing the mixture through reaction using a standard method, if necessary, in an appropriate solvent. For example, the adhesive setting resin can be obtained by thermally polymerizing the mixture at approximately 80°C for 8 hours using a polymerization initiator such as 2,2'-azobisisobutyronitrile in ethanol. The obtained polymer can be purified appropriately for use.

The amount of the adhesive setting resin incorporated in the hair cosmetic of the present invention can vary depending on the product form thereof and is generally 0.1 to 30% by mass, preferably 1 to 20% by mass, more preferably 2 to 15% by mass. If the amount is less than 0.1% by mass, the ability to arrange hair may be insufficient. If the adhesive setting resin is incorporated in an amount exceeding 30% by mass, the resulting hair cosmetic may make hair bristly.

The hair cosmetic of the present invention comprises at least one selected from a cationic surfactant, a silicone derivative, and a polyhydric alcohol, in addition to the adhesive setting resin. The cationic surfactant, the silicone derivative, and the polyhydric alcohol in the present invention are not particularly limited and can be any of those conventionally used in cosmetics or the like. Specific examples thereof can include the followings:

Examples of the cationic surfactant include: alkyl trimethyl ammonium salts, for example, stearyl trimethyl ammonium chloride and lauryl trimethyl ammonium chloride; alkylpyridinium salts, for example, cetylpyridinium chloride; dialkyl dimethyl ammonium salts, for example, distearyl dimethyl ammonium chloride; dicocoylethylhydroxyethylammonium methosulfate; poly(N,N'-dimethyl-3,5-methylenepiperidinium) chloride; quaternary ammonium salts of alkyl; alkyl dimethylbenzyl ammonium salts; alkyl isoquinolinium salts; dialkyl morpholinium salts; POE-alkylamine; alkylamine salts; polyamine fatty acid derivatives; amyl alcohol fatty acid derivatives; benzalkonium chloride; and benzethonium chloride.

The amount of the cationic surfactant incorporated in the hair cosmetic of the present invention is generally 0.1 to 10% by mass, preferably 0.2 to 5.0% by mass, more preferably 0.3 to 3.0% by mass. If the amount is less than

0.1% by mass, the resulting hair cosmetic may not impart smoothness to hair. If the cationic surfactant is incorporated in an amount exceeding 10% by mass, a problem in usability, such as stickiness, may arise.

The silicone derivative is preferably dimethicone copolyol (polyether-modified silicone) that is uniformly dissolved in a water/alcohol system or an alcohol system. Examples thereof can include: Silicone SC1014M and Silicone KF-6017P (both from Shin-Etsu Chemical Co., Ltd.); SILWET-10E and SILWET-10P (both from Nippon Unicar Co., Ltd.); and Silicone SH-3771 (Dow Corning Toray Co., Ltd.).

The amount of the silicone derivative incorporated in the hair cosmetic of the present invention is preferably 0.1 to 10% by mass, more preferably 0.1 to 5.0% by mass, with respect to the hair cosmetic. If the amount is less than 0.1% by mass, the resulting hair cosmetic may not impart smoothness to hair. If the silicone derivative is incorporated in an amount exceeding 10% by mass, a problem in usability, such as stickiness, may arise.

Examples of the polyhydric alcohol include: dihydric alcohols, for example, ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol; trihydric alcohols, for example, glycerin and trimethylolpropane; tetrahydric alcohols, for example, pentaerythritol such as 1,2,6-hexanetriol; pentahydric alcohols, for example, xylitol; hexahydric alcohols, for example, sorbitol and mannitol; polyhydric alcohol polymers, for example, diethylene glycol, dipropylene glycol, triethylene glycol, polypropylene glycol, tetraethylene glycol, diglycerin, polyethylene glycol, triglycerin, tetraglycerin, and polyglycerin; dihydric alcohol alkyl ethers, for example, ethylene glycol monomethyl ether, ethylene glycol monoethyl ether, ethylene glycol mono butyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether; dihydric alcohol alkyl ethers, for example, diethylene glycol monomethyl ether, diethylene glycol monoethyl ether, diethylene glycol mono butyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether; dihydric alcohol ether esters, for example, ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate; glycerin monoalkyl ethers, for example, chimyl alcohol, selachyl alcohol, and batyl alcohol; sugar alcohols, for example, sorbitol, maltitol, maltotriose, mannitol, sucrose, erythritol, glucose, fructose, sugar degraded from starch, maltose, xylitol, and alcohol reduced from sugar degraded from starch); Glysolid; tetrahydrofurfuryl alcohol; POE-tetrahydrofurfuryl alcohol; POP-butyl ether; POP/POE-butyl ether; tripolyoxypropylene glycerin ether; POP-glycerin ether; POP-glycerin, ether phosphate; and POP/POE-pentane erythritol ether, and polyglycerin.

The amount of the polyhydric alcohol incorporated in the hair cosmetic of the present invention is 0.1 to 20% by mass, preferably 0.2 to 15% by mass, more preferably 0.3 to 10% by mass, with respect to the hair cosmetic. If the amount is less than 0.1% by mass, the resulting hair cosmetic may not impart smoothness to hair. If the polyhydric alcohol is incorporated in an amount exceeding 20% by mass, a problem in usability, such as stickiness, may arise.

The hair cosmetic of the present invention comprises alcohol in addition to the adhesive setting resin and at least one selected from a cationic surfactant, a silicone derivative, and a polyhydric alcohol.

One or two or more selected from alcohols generally used in cosmetics, such as ethanol, can be selected appropriately and used as the alcohol in the hair cosmetic of the present invention. The amount of the alcohol incorporated is not particularly limited and can vary depending on the form of the hair cosmetic. The alcohol is usually incorporated in an amount from the lower limit for use as a solvent in the adhesive setting resin to 80% by mass. Moreover, in some times, it is preferred that the amount of the alcohol incorporated should be adjusted according to the amount of water incorporated, in terms of controlling usability.

The hair cosmetic of the present invention comprises the novel adhesive setting resin and further comprises at least one selected from a cationic surfactant, a silicone derivative, and a polyhydric alcohol. As a result, the hair cosmetic of the present invention exerts the abilities to set and arrange hair and imparts excellent feelings in use. Its form can be provided as various forms such as hair liquids, hair foams, hair mousse, hair sprays, hair mists, hair gels, and hair waxes.

The hair cosmetic of the present invention may comprise, for example, other ingredients conventionally used in hair cosmetics such as water according to the form thereof, without impairing the effect of the present invention.

## Examples

Hereinafter, the present invention will be described in more detail with reference to specific examples. However,

the present invention is not intended to be limited to Examples below. Moreover, the amount of each ingredient incorporated in Examples, etc., below represents % by mass, unless otherwise specified.

(Production Examples and Comparative Production Examples)

Monomers were polymerized according to the composition shown in Table 1 below to prepare adhesive setting resins of the present invention (Production Examples 1 to 6), a monomer C-free resin of Comparative Production Example 1, and a monomer D-free resin of Comparative Production Example 2.
Specifically, a mixture of monomers mixed in the total amount of 100 parts was prepared in advance. 100 parts of ethanol were added to a 1-L five-neck flask equipped with a dropping funnel containing this mixture, a reflux condenser, a thermometer, a tube for nitrogen substitution, and a stirrer. At the point in time when the ethanol was in a reflux state (approximately 80˚C) by heating under nitrogen flow, 1 part of a polymerization initiator (2,2'-azobisisobutyronitrile) was added into this ethanol, and the mixture was continuously added dropwise thereto for 2 hours. Then, the mixture was left for 8 hours in a reflux state to allow polymerization reaction to proceed. Next, the solvent was distilled off from the solution in the five-neck flask, and ethanol was added such that the solvent content in this solution was adjusted to obtain a hair cosmetic base solution having a 50% solid content.
[Table 1]

| Classification | Chemical structure (trade name) | Manufacturer | Production Example 1 | Production Example 2 | Production Example 3 | Production Example 4 | Production Example 5 | Production Example 6 | Comparative Production Example 1 | Comparative Production Example 2 |
|---|---|---|---|---|---|---|---|---|---|---|
| A | Butyl acrylate | Idemitsu Kosan Co., Ltd. | 40 | 35 | 30 | 15 | | | | 40 |
| A | Ethyl acrylate | TOAGOSEI CO., LTD. | | | | | 30 | | 30 | |
| A | Stearyl methacrylate (BLEMMER SMA) | NOF CORP. | | | | | | | | |
| A | Hydroxyethyl acrylate (HEA) | OSAKA ORGANIC CHEMICAL INDUSTRY LTD. | | | | 15 | | | 30 | |
| A | Methoxyethyl acrylate (Acrix C-1) | TOAGOSEI CO., LTD. | | | | | 15 | | | |
| B | Dimethylacrylamide (DMAA) | KOHJIN CHEMICAL CO., LTD. | | 40 | 30 | 30 | | 40 | 35 | 30 |
| B | Dimethylaminopropylacrylamide (DMAPAA) | TOAGOSEI CO., LTD. | | | | | 20 | | | |
| C | Polyoxyethylene glycol acrylate (n=10) (BLEMMER AE-400) | NOF CORP. | 55 | 20 | 15 | 15 | | | | 30 |
| C | Polyoxypropylene glycol acrylate (n=6) (BLEMMER AP-400) | NOF CORP. | | | 20 | 20 | 30 | 55 | | |
| D | Polyoxyethylene glycol diacrylate (n=23) (NK ESTER A-1000) | Shin-Nakamura Chemical Co. | 5 | 5 | 5 | 5 | | | 5 | |
| D | Polyoxyethylene glycol dimethacrylate (n=14) (NK ESTER 14G) | Shin-Nakamura Chemical Co. | | | | | | 5 | | |
| D | Glycerin dimethacrylate (BLEMMER NDMA) | NOF CORP. | | | | | 5 | | | |

(Examples and Comparative Examples)

The resins of Production Examples and Comparative Production Examples were used to prepare samples. The samples were evaluated for the ability to arrange hair, the ability to set hair, the ability to restyle hair, the smoothness of hair, and the moist feeling of hair in use.

Evaluation methods and evaluation criteria for each property are shown below.

1. Ability to arrange hair

0.5 g of the sample was applied to one bundle of black virgin hair (length: 20 cm, mass: 2 g). After drying at room temperature, the hair bundle was evaluated for ease of arrangement in a sensory test by 10 female expert panelists.

<Criteria of evaluation scores>

5: The hair was considerably easily arranged.
4: The hair was slightly easily arranged.
3: Normal.
2: The hair was not much easily arranged.
1: The hair was difficult to arrange.

<Evaluation Criteria>

◎: The total score was 40 or more.
○: The total score was 30 or more and less than 40.
△: The total score was 20 or more and less than 30.
×: The total score was less than 20.

2. Ability to set hair

0.4 g of the sample was applied to black virgin hair (length: 15 cm, weight: 1 g), then spread over the hair using a comb, and styled such that the hair became straight. Five strands were prepared per sample. These strands were dried at 50°C for 1 hour and then hung on a graduated board. A length (b) of each bent strand was measured in a thermo-hygrostat with a temperature of 30°C and a humidity of 90% RH. The ability to set hair (ability to keep hairstyles) was determined according to a formula shown below using a length (a) of the bent strand measured in advance before application of the sample. A numeric value closer to 100% represents the higher ability to set hair and more excellent moisture resistance.

$$\text{Ability to keep hairstyles (\%)} = \{(a-b)/a\} \times 100$$

<Evaluation Criteria>

◎: The value was 90% or more.
○: The value was 70% to less than 90%.
△: The value was 50% to less than 70%.
×: The value was less than 50%.

3. Ability to restyle hair

0.5 g of the sample was applied to one bundle of black virgin hair (length: 20 cm, mass: 2 g). After drying at room temperature, the hair bundle was evaluated for ease of restyling (ability to restyle hair) in a sensory test by 10 female expert panelists when the hair was styled immediately after the application and restyled one hour thereafter.

<Criteria of evaluation scores>

5: Considerable ability to restyle hair.
4: Slight ability to restyle hair.
3: Normal.
2: Not much ability to restyle hair.
1: No ability to restyle hair.

<Evaluation Criteria>

◎: The total score was 40 or more.
○: The total score was 30 or more and less than 40.
△: The total score was 20 or more and less than 30.
×: The total score was less than 20.

4. Smoothness of hair

0.5 g of the sample was applied to one bundle of black virgin hair (length: 20 cm, mass: 2 g). After drying at room temperature, the hair bundle was evaluated for the smoothness of the hair in a sensory test by 10 female expert panelists.

<Criteria of evaluation scores>

5: The hair felt considerably smooth.
4: The hair felt slightly smooth.
3: Normal.
2: The hair did not feel much smooth.
1: The hair did not feel smooth.

<Evaluation Criteria>

◎: The total score was 40 or more.
○: The total score was 30 or more and less than 40.
△: The total score was 20 or more and less than 30.
×: The total score was less than 20.

5. Moist feeling

0.5 g of the sample was applied to one bundle of black virgin hair (length: 20 cm, mass: 2 g). After drying at room temperature, the hair bundle was evaluated for the moist feeling of the hair in a sensory test by 10 female expert panelists.

<Criteria of evaluation scores>

5: The hair felt considerably moist.
4: The hair felt slightly moist.
3: Normal.
2: The hair did not feel much moist.
1: The hair did not feel moist.

<Evaluation Criteria>

◎: The total score was 40 or more.
○: The total score was 30 or more and less than 40.
△: The total score was 20 or more and less than 30.
×: The total score was less than 20.

(1) In the case of using cationic surfactant

Samples were prepared according to the composition listed in Table 2 below. The samples were prepared as liquid hair-styling agents by adding (3) to (2), stirring the mixture, then adding (1) thereto, further stirring the mixture, then adding (4) thereto, and stirring the mixture. Subsequently, the properties of each sample were evaluated according to the criteria described above. The results are also shown in Table 2.

[Table 2]

| | Comparative Example 1 | Comparative Example 2 | Example 1 | Example 2 | Example 3 | Example 4 |
|---|---|---|---|---|---|---|
| (1) Water | 64.1 | 60 | 64 | 63.1 | 59.1 | 54.1 |
| (2) Ethanol | 35 | 35 | 35 | 35 | 35 | 35 |
| (3) Stearyl trimonium chloride | 0.9 | | 0.9 | 0.9 | 0.9 | 0.9 |
| (4) Production Example 3 | | 5 | 0.1 | 1 | 5 | 10 |
| Total | 100 | 100 | 100 | 100 | 100 | 100 |
| | | | | | | |
| Ability to arrange hair | × | ◎ | Δ | ○ | ◎ | ◎ |
| Ability to set hair | × | ○ | Δ | ○ | ○ | ○ |
| Ability to restyle hair | × | ○ | Δ | ○ | ○ | ◎ |
| Smoothness of hair | ◎ | Δ | ◎ | ◎ | ◎ | ◎ |

As is evident from the results shown in Table 2, the adhesive setting resin (Production Example 3)-free sample (Comparative Example 1) was much inferior in the ability to arrange hair, the ability to set hair, and the ability to restyle hair. Moreover, the samples comprising both the adhesive setting resin and the cationic surfactant (stearyl trimonium chloride) (Examples 1 to 4) were improved in the smoothness of hair, compared with the cationic surfactant-free sample (Comparative Example 2).

Samples were prepared according to the composition listed in Table 3 below, and the properties of each sample were evaluated according to the criteria described above. The results are also shown in Table 3.

[Table 3]

| | Comparative Example 1 | Comparative Example 3 | Comparative Example 4 | Example 5 | Example 6 | Example 3 | Example 7 | Example 8 | Example 9 |
|---|---|---|---|---|---|---|---|---|---|
| (1) Water | 64.1 | 59.1 | 59.1 | 59.1 | 59.1 | 59.1 | 59.1 | 59.1 | 59.1 |
| (2) Ethanol | 35 | 35 | 35 | 35 | 35 | 35 | 35 | 35 | 35 |
| (3) Stearyl trimonium chloride | 0.9 | 0.9 | 0.9 | 0.9 | 0.9 | 0.9 | 0.9 | 0.9 | 0.9 |
| (4) Comparative Production Example1 | | 5 | | | | | | | |
| (4) Comparative Production Example2 | | | 5 | | | | | | |
| (4) Production Example 1 | | | | 5 | | | | | |
| (4) Production Example 2 | | | | | 5 | | | | |
| (4) Production Example 3 | | | | | | 5 | | | |
| (4) Production Example 4 | | | | | | | 5 | | |
| (4) Production Example 5 | | | | | | | | 5 | |
| (4) Production Example 6 | | | | | | | | | 5 |
| Total | 100 | 100 | 100 | 100 | 100 | 100 | 100 | 100 | 100 |
| | | | | | | | | | |
| Ability to arrange hair | × | Δ | Δ | ○ | ◎ | ◎ | ◎ | ○ | ○ |
| Ability to set hair | × | Δ | Δ | ○ | ○ | ○ | ○ | ○ | ○ |

(continued)

| | Comparative Example 1 | Comparative Example 3 | Comparative Example 4 | Example 5 | Example 6 | Example 3 | Example 7 | Example 8 | Example 9 |
|---|---|---|---|---|---|---|---|---|---|
| Ability to restyle hair | × | Δ | Δ | ○ | ○ | ○ | ○ | ○ | ○ |
| Smoothness of hair | ◎ | ◎ | ◎ | ◎ | ◎ | ◎ | ◎ | ◎ | ◎ |

The adhesive setting resin (Production Examples 1 to 6)-free sample (Comparative Example 1) and the samples (Comparative Examples 3 and 4) comprising the monomer Cor D-free resin (Comparative Production Example 1 or 2) failed to produce the sufficient abilities to arrange, set, and restyle hair.

(2) In the case of using silicone derivative

Samples were prepared according to the composition listed in Table 4 below. The samples were prepared by adding (3) to a mixed solution of (1) and (2), stirring the mixture, further adding (4) thereto, and stirring the mixture. Subsequently, the properties of each sample were evaluated according to the criteria described above. The results are also shown in Table 4.

[Table 4]

| | Comparative Example 5 | Comparative Example 6 | Example 10 | Example 11 | Example 12 | Example 13 |
|---|---|---|---|---|---|---|
| (1) Water | 64 | 60 | 63.9 | 63 | 59 | 54 |
| (2) Ethanol | 35 | 35 | 35 | 35 | 35 | 35 |
| (3) PEG-10 methyl ether dimethicone (SILWET-10E) | 1 | | | 1 | 1 | 1 |
| (4) Production Example 3 | | 5 | 0.1 | 1 | 5 | 10 |
| Total | 100 | 100 | 100 | 100 | 100 | 100 |
| | | | | | | |
| Ability to arrange hair | × | ◎ | Δ | ○ | ◎ | ◎ |
| Ability to set hair | × | ○ | Δ | ○ | ○ | ○ |
| Ability to restyle hair | × | ○ | Δ | ○ | ○ | ◎ |
| Smoothness of hair | ◎ | △ | ◎ | ◎ | ◎ | ◎ |

As is evident from the results shown in Table 4, the adhesive setting resin (Production Example 3)-free sample (Comparative Example 5) was inferior in the ability to arrange hair, the ability to set hair, and the ability to restyle hair. The silicone derivative (PEG-10 methyl ether dimethicone)-free sample (Comparative Example 6) imparted a feeling in use that lacked the smoothness of hair.

Samples were prepared according to the composition listed in Table 5 below, and the properties of each sample were evaluated according to the criteria described above. The results are also shown in Table 5.

[Table 5]

| | Comparative Example 5 | Comparative Example 7 | Comparative Example 8 | Example 14 | Example 15 | Example 12 | Example 16 | Example 17 | Example 18 |
|---|---|---|---|---|---|---|---|---|---|
| (1) Water | 64 | 59 | 59 | 59 | 59 | 59 | 59 | 59 | 59 |
| (2) Ethanol | 35 | 35 | 35 | 35 | 35 | 35 | 35 | 35 | 35 |
| (3) PEG-10 methyl ether dimethicone | 1 | 1 | 1 | 1 | 1 | 1 | 1 | 1 | 1 |
| (4) Comparative Production Example1 | | 5 | | | | | | | |
| (4) Comparative Production Example2 | | | 5 | | | | | | |
| (4) Production Example 1 | | | | 5 | | | | | |
| (4) Production Example 2 | | | | | 5 | | | | |
| (4) Production Example 3 | | | | | | 5 | | | |
| (4) Production Example 4 | | | | | | | 5 | | |
| (4) Production Example 5 | | | | | | | | 5 | |
| (4) Production Example 6 | | | | | | | | | 5 |
| Total | 100 | 100 | 100 | 100 | 100 | 100 | 100 | 100 | 100 |
| | | | | | | | | | |
| Ability to arrange hair | × | Δ | Δ | ○ | ◎ | ◎ | ◎ | ○ | ○ |
| Ability to set hair | × | Δ | Δ | ○ | ○ | ○ | ○ | ○ | ○ |

(continued)

| | Comparative Example 5 | Comparative Example 7 | Comparative Example 8 | Example 14 | Example 15 | Example 12 | Example 16 | Example 17 | Example 18 |
|---|---|---|---|---|---|---|---|---|---|
| Ability to restyle hair | × | Δ | Δ | ○ | ○ | ○ | ○ | ○ | ○ |
| Smoothness of hair | ◎ | ◎ | ◎ | ◎ | ◎ | ◎ | ◎ | ◎ | ◎ |

The adhesive setting resin (Production Examples 1 to 6)-free sample (Comparative Example 5) and the samples (Examples 7 and 8) comprising the monomer C- or D-free resin (Comparative Production Example 1 or 2) failed to exert the sufficient abilities to arrange, set, and restyle hair.

(3) In the case of using a polyhydric alcohol

Samples were prepared according to the composition listed in Tables 6 and 7 below. The samples were prepared by adding (3) to a mixed solution of (1) and (2), stirring the mixture, further adding (4) thereto, and stirring the mixture. Subsequently, the properties of each sample were evaluated according to the criteria described above. The results are also shown in Tables 6 and 7.

[Table 6]

| | Comparative Example 9 | Comparative Example 2 | Example 19 | Example 20 | Example 21 | Example 22 |
|---|---|---|---|---|---|---|
| (1) Water | 60 | 60 | 59.9 | 59 | 55 | 50 |
| (2) Ethanol | 35 | 35 | 35 | 35 | 35 | 35 |
| (3) Butylene glycol | 5 | | 5 | 5 | 5 | 5 |
| (4) Production Example 3 | | 5 | 0.1 | 1 | 5 | 10 |
| Total | 100 | 100 | 100 | 100 | 100 | 100 |
| | | | | | | |
| Ability to arrange hair | × | ◎ | Δ | ○ | ◎ | ◎ |
| Ability to set hair | × | ○ | Δ | ○ | ○ | ○ |
| Ability to restyle hair | × | ○ | Δ | ○ | ○ | ◎ |
| Moist feeling of hair | ○ | Δ | ○ | ○ | ○ | ○ |

[Table 7]

| | Comparative Example 10 | Comparative Example 2 | Example 23 | Example 24 | Example 25 | Example 26 |
|---|---|---|---|---|---|---|
| (1) Water | 60 | 60 | 59.9 | 59 | 55 | 50 |
| (2) Ethanol | 35 | 35 | 35 | 35 | 35 | 35 |
| (3)Diglycerin | 5 | | 5 | 5 | 5 | 5 |
| (4) Production Example 3 | | 5 | 0.1 | 1 | 5 | 10 |
| Total | 100 | 100 | 100 | 100 | 100 | 100 |
| | | | | | | |
| Ability to arrange hair | × | ◎ | Δ | ○ | ◎ | ◎ |
| Ability to set hair | × | ○ | Δ | ○ | ○ | ○ |
| Ability to restyle hair | × | ○ | Δ | ○ | ○ | ◎ |

(continued)

| | Comparative Example 10 | Comparative Example 2 | Example 23 | Example 24 | Example 25 | Example 26 |
|---|---|---|---|---|---|---|
| Moist feeling of hair | ○ | Δ | ○ | ○ | ○ | ○ |

As is evident from the results shown in Tables 6 and 7, the adhesive setting resin (Production Example 3)-free samples (Comparative Examples 9 and 10) were inferior in the ability to arrange hair, the ability to set hair, and the ability to restyle hair. The polyhydric alcohol (butylene glycol or diglycerin)-free samples (Comparative Example 2) was inferior in a feeling in use (moist feeling of hair).

Samples were prepared according to the composition listed in Tables 8 and 9 below, and the properties of each sample were evaluated according to the criteria described above. The results are also shown in Tables 8 and 9.

[Table 8]

| | Comparative Example 9 | Comparative Example 11 | Comparative Example 12 | Example 27 | Example 28 | Example 21 | Example 29 | Example 30 | Example 31 |
|---|---|---|---|---|---|---|---|---|---|
| (1) Water | 60 | 55 | 55 | 55 | 55 | 55 | 55 | 55 | 55 |
| (2) Ethanol | 35 | 35 | 35 | 35 | 35 | 35 | 35 | 35 | 35 |
| (3) Butylene glycol | 5 | 5 | 5 | 5 | 5 | 5 | 5 | 5 | 5 |
| (4) Comparative Production Example1 | | 5 | | | | | | | |
| (4) Comparative Production Example2 | | | 5 | | | | | | |
| (4) Production Example 1 | | | | 5 | | | | | |
| (4) Production Example 2 | | | | | 5 | | | | |
| (4) Production Example 3 | | | | | | 5 | | | |
| Production Example 4 | | | | | | | | | |
| (4) Production Examples 5 | | | | | | | | 5 | |
| (4) Production Example 6 | | | | | | | | | 5 |
| Total | 100 | 100 | 100 | 100 | 100 | 100 | 100 | 100 | 100 |
| | | | | | | | | | |
| Ability to arrange hair | × | Δ | Δ | ○ | ◎ | ◎ | ◎ | ○ | ○ |
| Ability to set hair | × | Δ | Δ | ○ | ○ | ○ | ○ | ○ | ○ |

(continued)

| | Comparative Example 9 | Comparative Example 11 | Comparative Example 12 | Example 27 | Example 28 | Example 21 | Example 29 | Example 30 | Example 31 |
|---|---|---|---|---|---|---|---|---|---|
| Ability to restyle hair | × | Δ | Δ | ○ | ○ | ○ | ○ | ○ | ○ |
| Moist feeling of hair | ○ | ○ | ○ | ○ | ○ | ○ | ○ | ○ | ○ |

[Table 9]

| | Comparative Example 10 | Comparative Example 13 | Comparative Example 14 | Example 32 | Example 33 | Example 25 | Example 34 | Example 35 | Example 36 |
|---|---|---|---|---|---|---|---|---|---|
| (1) Water | 60 | 55 | 55 | 55 | 55 | 55 | 55 | 55 | 55 |
| (2) Ethanol | 35 | 35 | 35 | 35 | 35 | 35 | 35 | 35 | 35 |
| (3) Diglycerin | 5 | 5 | 5 | 5 | 5 | 5 | 5 | 5 | 5 |
| (4) Comparative Production Example1 | | 5 | | | | | | | |
| (4) Comparative Production Example2 | | | 5 | | | | | | |
| (4) Production Example 1 | | | | 5 | | | | | |
| (4) Production Example 2 | | | | | 5 | | | | |
| (4) Production Example 3 | | | | | | 5 | | | |
| z Production Example 4 | | | | | | | 5 | | |
| (4) Production Example 5 | | | | | | | | 5 | |
| (4) Production Example 6 | | | | | | | | | 5 |
| Total | 100 | 100 | 100 | 100 | 100 | 100 | 100 | 100 | 100 |
| | | | | | | | | | |
| Ability to arrange hair | × | Δ | Δ | ○ | ◎ | ◎ | ◎ | ○ | ○ |
| Ability to set hair | × | Δ | Δ | ○ | ○ | ○ | ○ | ○ | ○ |
| Ability to restyle hair | × | Δ | Δ | ○ | ○ | ○ | ○ | ○ | ○ |

(continued)

| | Comparative Example 10 | Comparative Example 13 | Comparative Example 14 | Example 32 | Example 33 | Example 25 | Example 34 | Example 35 | Example 36 |
|---|---|---|---|---|---|---|---|---|---|
| Moist feeling of hair | ○ | ○ | ○ | ○ | ○ | ○ | ○ | ○ | ○ |

The adhesive setting resin (Production Examples 1 to 6)-free sample (Comparative Examples 9 and 10) and the samples (Examples 11, 12, 13, and 14) comprising the monomer C- or D-free resin (Comparative Production Example 1 or 2) failed to exert the sufficient abilities to arrange and restyle hair.

The other Examples are shown below.

(Example 37)

Liquid styling agent

| | | |
|---|---|---|
| (1) | ion-exchanged water | balance |
| (2) | Peg-6 | 5 |
| (3) | PEG-8 | 5 |
| (4) | PEG-32 | 5 |
| (5) | glycerin | 5 |
| (6) | ethanol | 35 |
| (7) | fragrance | q.s. |
| (8) | polymer obtained in Production Example 3 | 5 |
| (9) | (alkyl acrylate/diacetone acrylamide) copolymer | 2.5 |
| (10) | citric acid | q.s. |

<Production Process>

Water-soluble ingredients (2) to (5) were added to water (1) and dissolved by stirring to prepare aqueous parts. Next, (7) was added to (6), and the mixture was stirred for solubilization. Then, (8) and (9) were added thereto, and the mixture was stirred to prepare alcohol parts. The aqueous parts and the alcohol parts were mixed, and (10) was added to the mixture to obtain a liquid styling agent.

(Example 38)

Liquid styling agent

| | | |
|---|---|---|
| (1) | Ion-exchanged water | balance |
| (2) | DPG | 5 |
| (3) | Glycerin | 0.5 |
| (4) | PEG-6 | 5 |
| (5) | PEG-32 | 5 |
| (6) | Ethanol | 35 |
| (7) | Fragrance | q.s. |
| (8) | Polymer obtained in Production Example | 3 5 |
| (9) | Stearyl trimonium chloride | 0.5 |
| (10) | EDTA-2Na | q.s. |
| (11) | Sodium lactate | q.s. |

<Production Process>

Water-soluble ingredients (2) to (5) and (11) were added to water (1) and dissolved by stirring to prepare aqueous parts. Next, (7) was added to (6), and the mixture was stirred for solubilization. Then, (8) and (9) were added thereto, and the mixture was stirred to prepare alcohol parts. The aqueous parts and the alcohol parts were mixed, and (10) was added to the mixture to obtain a liquid styling agent.

(Example 39)

Liquid styling agent

| | | |
|---|---|---|
| (1) | Ethanol | balance |
| (2) | Polymer obtained in Production Example 3 | 5 |
| (3) | Hydrogenated polyisobutene | 5 |
| (4) | PEG/PPG-19/19 dimethicone | 5 |
| (5) | Dimethicone | 3 |
| (6) | PPG-70 polyglyceryl-10 | 6 |
| (7) | Fragrance | q.s. |

<Production Process>

(7) was added to (1) and stirred for solubilization. Then, (2) to (6) were sequentially added thereto, and the mixture was well stirred to obtain a liquid styling agent.

(Example 40)

Hair-styling gel

| | | |
|---|---|---|
| (1) | carboxyvinyl polymer | 0.7 |
| (2) | polymer obtained in Production Example 3 | 5.0 |
| (3) | glycerin | 2.5 |
| (4) | 1,3-butylene glycol | 2.5 |
| (5) | polyoxyethylene octyldodecyl ether (20EO) | 0.5 |
| (6) | polyether-modified dimethylpolysiloxane | 1.0 |
| (7) | sodium hydroxide (adjusted to pH 7.5) | q.s. |
| (8) | ethanol | 20.0 |
| (9) | polyoxyethylene octyldodecyl ether | 0.1 |
| (10) | fragrance | 0.1 |
| (11) | trisodium edetate | 0.03 |
| (12) | ion-exchanged water | balance |
| (13) | (alkyl acrylate/diacetone acrylamide) copolymer | 5.0 |

<Production Process>

(6) was added to (3), (4), (5), and a portion of (12), and the mixture was emulsified using a homo mixer. Subsequently, a portion of the remaining (12) was added to the emulsion to prepare emulsified parts. On the other hand, (1), (2), (7), (8), (9), (10), (11), and (13) were uniformly dissolved in the remaining portion of (12). To this solution, the emulsified parts were added to obtain a hair-styling gel emulsion.

(Example 41)

Hair-styling gel

| | | |
|---|---|---|
| (1) | Hydroxyethylcellulose | 0.7 |
| (2) | Polymer obtained in Production Example 3 | 12.0 |

(continued)

| | | |
|---|---|---|
| (3) | Propylene glycol | 2.5 |
| (4) | 1,3-Butylene glycol | 2.5 |
| (5) | Polyoxyethylene hydrogenated castor oil (40EO) | 0.5 |
| (6) | Amino-modified high-molecular-weight silicone | 1.0 |
| (7) | Sodium hydroxide (adjusted to pH 7.5) | q.s. |
| (8) | Ethanol | 20.0 |
| (9) | Polyoxyethylene octyldodecyl ether | 0.1 |
| (10) | Fragrance | 0.1 |
| (11) | Trisodium edetate | 0.03 |
| (12) | Ion-exchanged water | balance |
| (13) | (octylacrylamide/hydroxypropyl acrylate/butylaminoethyl methacrylate) copolymer | 16.0 |

<Production Process>

A hair-styling gel was produced according to Example 40.

(Example 42)

Hair-styling gel

| | | |
|---|---|---|
| (1) | (PEG-240/decyltetradeceth-20/HDI) copolymer | 2.0 |
| (2) | polymer obtained in Production Example 3 | 1.0 |
| (3) | diglycerin | 2.5 |
| (4) | polyethylene glycol 1000 | 2.5 |
| (5) | polyoxyethylene hydrogenated castor oil (40EO) | 0.5 |
| (6) ends | dimethylpolysiloxane modified with hydroxy at both (1,000,000 mPa·s) | 1.0 |
| (7) | sodium hydroxide (adjusted to pH 7.5) | q.s. |
| (8) | ethanol | 20.0 |
| (9) | polyoxyethylene octyldodecyl ether | 0.1 |
| (10) | fragrance | 0.1 |
| (11) | trisodium edetate | 0.03 |
| (12) | ion-exchanged water | balance |
| (13) | (alkyl acrylate/diacetone acrylamide) copolymer | 1.0 |

<Production Process>

A hair-styling gel was produced according to Example 40.

(Example 43)

Hair wax

| | | |
|---|---|---|
| (1) | methylpolysiloxane | 2.0 |
| (2) | microcrystalline wax | 12.0 |
| (3) | liquid paraffin | 3.5 |
| (4) | hydrogenated polyisobutene | 3.5 |
| (5) | pentaerythrityl tetra-2-ethylhexanoate | 3.0 |
| (6) | PEG-60 glyceryl isostearate | 1.0 |

(continued)

| | | |
|---|---|---|
| (7) | glyceryl stearate | 1.0 |
| (8) | deodorized cetanol (derived from plant oil) | 3.3 |
| (9) | stearyl alcohol | 0.9 |
| (10) | tocopherol | 0.5 |
| (11) | fragrance | 0.1 |
| (12) | ion-exchanged water | balance |
| (13) | propylene glycol | 8.0 |
| (14) | stearyl trimonium chloride | 1.2 |
| (15) | kaolin | 2.5 |
| (16) | triethanolamine | 0.4 |
| (17) | polyvinylpyrrolidone-vinyl acetate copolymer | 1.8 |
| (18) | polymer obtained in Production Example 3 | 5.0 |

<Production Process>

(1) to (11) were dissolved by stirring at 85˚C (oil-phase parts). On the other hand, (12) to (14) were dissolved by stirring at 75˚C (aqueous-phase parts). The oil-phase parts were added to the aqueous-phase parts, and the mixture was emulsified. Then, (15) was added to the emulsion. Subsequently, after neutralization by the addition of (16), (17) and (18) were added thereto, and the mixture was degassed and cooled.

(Example 44)

Hair water

| | | |
|---|---|---|
| (1) | Ion-exchanged water | balance |
| (2) | Ethanol | 35.0 |
| (3) | Glycerin | 2.0 |
| (4) | PG | 1.0 |
| (5) | Stearyl trimonium chloride | 0.9 |
| (6) | Sodium lactate | q.s. |
| (7) | PEG/PPG-14/7 dimethyl ether | 2.0 |
| (8) | Fragrance | q.s. |
| (9) | Polymer obtained in Production Example 3 | 1.0 |

<Production Process>

(3) and (4) were added to water (1) and dissolved by stirring to prepare aqueous parts. Next, (8) was added to (2), and the mixture was stirred for solubilization. Then, (5), (7), and (9) were added thereto, and the mixture was stirred to prepare alcohol parts. The aqueous parts and the alcohol parts were mixed, and (6) was added to the mixture to obtain hair water.

(Example 45)

Out-bath treatment

| | | |
|---|---|---|
| (1) | Volatile isoparaffin | 10 |
| (2) | Dimethylpolysiloxane | 1 |
| (3) | Ethanol | 10 |
| (4) | 1,3-Butylene glycol | 5 |

(continued)

| | | |
|---|---|---|
| (5) | Isostearic acid | 0.5 |
| (6) | Polyoxyethylene hydrogenated castor oil | 0.1 |
| (7) | 2-Alkyl-N-carboxymethyl-N-hydroxyethyl imidazolinium betaine | 1 |
| (8) | Sodium hydroxide | 0.3 |
| (9) | Highly polymerized methylpolysiloxane | 2 |
| (10) | Carboxyvinyl polymer | 0.8 |
| (11) | Phenoxy ethanol | q.s. |
| (12) | EDTA-2Na | q.s. |
| (13) | Polymer obtained in Production Example 3 | 0.5 |
| (14) | Purified water | balance |
| (15) | Fragrance | q.s. |

<Production Process>

(10) and (12) were added to (14) and stirred to prepare aqueous parts. Next, (15) and (11) were added to (3), and the mixture was well stirred to prepare alcohol parts, which were then added to the aqueous parts. (6) was added to (4), and the mixture was stirred. Then, a mixture of (2), (5), and (9) was gradually added thereto, and the mixture was treated with a homo mixer. Further, (7) and a portion of water (14) were added thereto, and the mixture was treated again with a homo mixer to prepare emulsified parts. The emulsified parts were added to the aqueous/alcohol parts, and the mixture was well stirred and then neutralized using (8). Finally, (13) was added thereto to obtain the out-bath treatment of interest.

## Claims

**1.** A hair cosmetic comprising:

(a) an adhesive setting resin obtained by polymerizing: at least one monomer represented by the following formula (A):

[Formula 1]

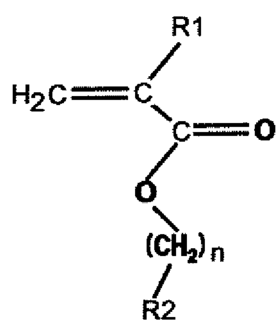

(A)

wherein R1 represents H or $CH_3$; n represents an integer of 0 to 30; $(CH_2)_n$ contains a branched chain; and R2 represents H, OH, $OCH_3$ , $OCH_2CH_3$, or phenyl,
and/or
at least one monomer represented by the following formula (B):

[Formula 2]

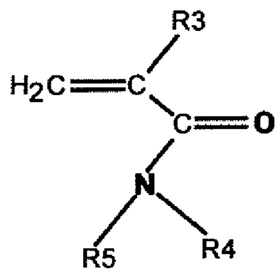

(B)

wherein R3 represents H or $CH_3$; R4 and R5, which may be the same or different, each represent H or $(CH_2)_1R'$; 1 represents an integer of 1 to 3; R' represents H, OH, or -NR"R'"; and R" and R'", which may be the same or different, each represent H or a C1-C3 alkyl group,
and
at least one monomer represented by the following formula (C) :

[Formula 3]

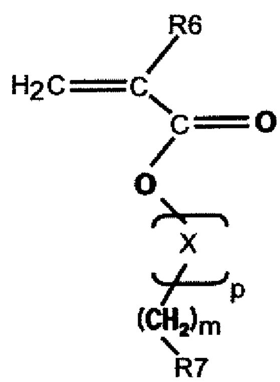

(C)

wherein R6 represents H or $CH_3$; p represents an integer of 1 to 100; m represents an integer of 0 to 30; R7 represents H, OH, $OCH_3$, $OCH_2CH_3$, or phenyl; and X represents an oxyethylene group (EO), an oxypropylene group (PO), an oxybutylene group (BO), or glyceryl,
and
at least one monomer represented by the following formula D:

[Formula 4]

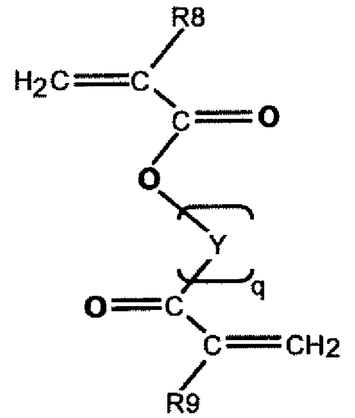

(D)

wherein R8 represents H or $CH_3$; q represents an integer of 1 to 100; and Y represents an oxyethylene group (EO), an oxypropylene group (PO), an oxybutylene group (BO), a linear or branched oxyalkylene group having 5 or more carbon atoms, or glyceryl, provided that q represents 1 when Y represents a linear or branched oxyalkylene group having 5 or more carbon atoms,

(b) at least one selected from a cationic surfactant, a silicone derivative, and a polyhydric alcohol, and

(c) alcohol.

2. The hair cosmetic according to claim 1, wherein the adhesive setting resin has a structure represented by the following formula (I):

[Formula 5]

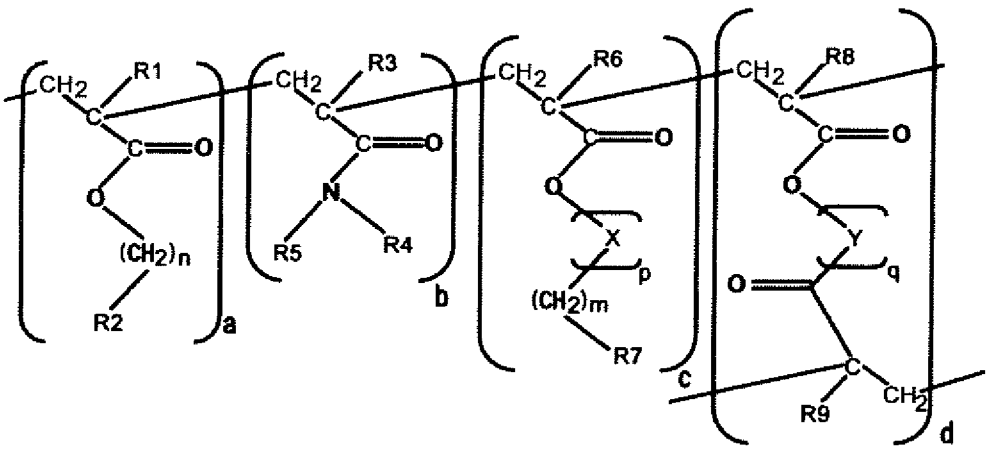

(I)

wherein R1 to R9, n, m, p, and q are as defined in the formulas A to D; a represents a number within the range of $40 < a < 400$; b represents a number within the range of $80 \leq b < 300$; c represents a number within the range of $30 < c < 300$; and d represents a number within the range of $0 < d < 10$.

3. The hair cosmetic according to claim 1 or 2, wherein the silicone derivative is a polyether-modified silicone.

**INTERNATIONAL SEARCH REPORT**

International application No.
PCT/JP2010/056058

A. CLASSIFICATION OF SUBJECT MATTER
*A61K8/34*(2006.01)i, *A61K8/81*(2006.01)i, *A61K8/894*(2006.01)i, *A61K8/898*(2006.01)i, *A61Q5/06*(2006.01)i, *C08F220/10*(2006.01)i, *C08F290/06*(2006.01)i

According to International Patent Classification (IPC) or to both national classification and IPC

B. FIELDS SEARCHED

Minimum documentation searched (classification system followed by classification symbols)
A61K8/34, A61K8/81, A61K8/894, A61K8/898, A61Q5/06, C08F220/10, C08F290/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2002-322219 A (Lion Corp.), 08 November 2002 (08.11.2002), claims; paragraphs [0047], [0052]; example 10 (Family: none) | 1-3 |

☐ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 May, 2010 (07.05.10) | 18 May, 2010 (18.05.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006213706 A
- JP 2003171244 A
- JP HEI1045546 A


### Non-patent literature cited in the description


- Functions of Hair-styling Agents and State-of-the-Art Technology. Development of Advanced Cosmetics II. CMC Publishing Co., Ltd, 1996